Europäisches Patentamt

European Patent Office

Office européen des brevets

(19)

(11) Publication number: **0 205 333 B1**

# EUROPEAN PATENT SPECIFICATION

(12)

(45) Date of publication of patent specification: **24.07.91**

(51) Int. Cl.⁵: **A61L 27/00, A61C 8/00, A61F 2/28, A61B 17/58**

(21) Application number: **86304388.1**

(22) Date of filing: **09.06.86**

The file contains technical information submitted after the application was filed and not included in this specification

(54) **Implant material.**

(30) Priority: **10.06.85 JP 125696/85**
        **20.09.85 JP 207836/85**

(43) Date of publication of application:
        **17.12.86 Bulletin 86/51**

(45) Publication of the grant of the patent:
        **24.07.91 Bulletin 91/30**

(84) Designated Contracting States:
        **DE FR GB IT NL**

(56) References cited:
        **DE-A- 3 005 264**
        **GB-A- 1 530 670**
        **US-A- 4 222 128**
        **US-A- 4 379 694**

(73) Proprietor: **KUREHA KAGAKU KOGYO KABUSHIKI KAISHA**
        **9-11 Horidome-cho 1-chome Nihonbashi Chuo-ku**
        **Tokyo 103(JP)**

(72) Inventor: **Nagai, Hirosi**
        **4-2-47 Fujimi-cho**
        **Chofu-shi Tokyo(JP)**

(74) Representative: **Woods, Geoffrey Corlett et al J.A. KEMP & CO. 14 South Square Gray's Inn London WC1R 5EU(GB)**

EP 0 205 333 B1

## Description

The present invention relates to an implant material.

In recent years, with the development of biotechnology, techniques which implant artificial material in a shape of joint or radix dentis into the bone tissue of a human body have been used.

As the artificial material, metallic materials such as alloys of cobalt and chromium, titanium and tantalum, and ceramic materials such as hydroxyapatite, zirconia, alumina and glassy carbon are known. However although the metallic materials have excellent mechanical strength, they have poor biocompatibility and some of them are injurious due to the eduction of metal ions. On the other hand, the ceramic materials have excellent biocompatibility, but they still have poor mechanical properties.

Thus conventional artificial materials have any one of the disadvantages of toxicity, compatibility to bones, mechanical properties or durability and accordingly they can not be said to be satisfactory. Therefore, trials combining different materials have been carried out.

For instance, JP-A-53/28997 discloses, as an implant material comprising a metallic material and another material integrated in one body, a material made by forming a melt-ejected layer of a powdery ceramic or a powdery hydroxyapatite on the surface of a metallic material. Such an implant material is expected to have the biocompatibility of hydroxyapatite and the mechanical strength of the metallic material. However, cracks are apt to form in the melt-ejected layer due to the differences of thermal expansion between the metallic material and the melt-ejected layer, and eduction of metal ions from the crack is feared.

JP-A-57/156757 discloses an implant material made by coating the outer surface of a metallic material with a composition singly formed from a thermoplastic polymer such as a polysulfone, high density polyethylene or poly(methyl methacrylate), or formed by adding from 20 to 30% by weight of an inorganic material such as calcium phosphate or hydroxyapatite to the thermoplastic polymer.

However, the thermoplastic polymers have insufficient adhesion to the metallic material, stability in a living body, osteogenesis and mechanical properties, and accordingly they are not practically reliable.

DE-A-3005264 relates to adhering a prosthesis to bone using a particular thermoplastic resin glue which is polymethylmethacrylate. US-A-4222128 relates to strengthening an implant material made of sintered apatite by filling or impregnating holes in the apatite with a thermoplastic or thermosetting resin.

We have sought an implant material which has excellent adhesion to the metallic material, osteogenesis, mechanical properties, feeling on actual use, and reliability on practical use.

The present invention provides an implant material suitable for use in a living body, which material comprises a metallic material covered by a layer comprising a biocompatible, stable thermoset resin.

The resin layer may further comprise hydroxyapatite.

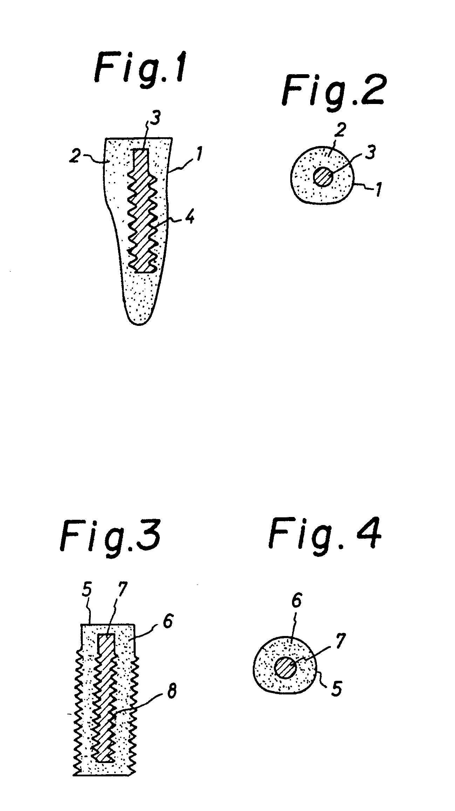

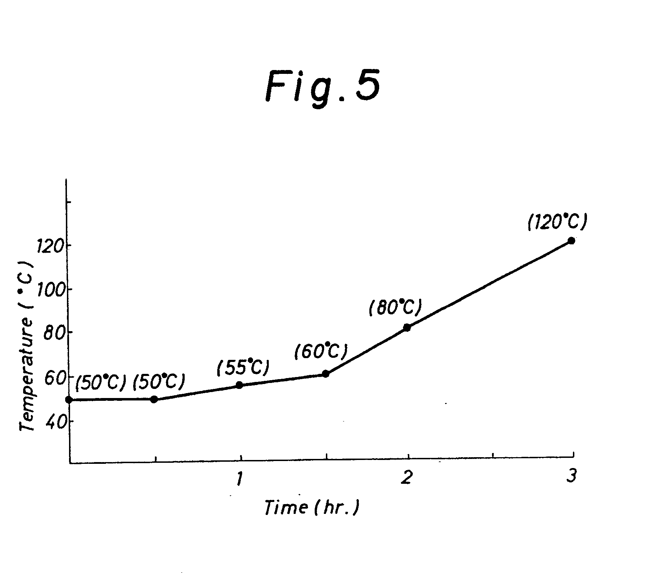

Of the attached drawings, Figs. 1 and 3 and Figs. 2 and 4 are respectively the vertical sectional view and the cross sectional view of implant materials according to the present invention. Fig. 5 is a relationship between temperature and time.

Although the thermoset resin has excellent adhesion to the metallic material, since it is implanted in living body for a long period of time, it is necessary that it has excellent biocompatibility, does not self-deteriorate and does not cause the collapse of the living cells. For instance a polymer of a monomer such as bisphenol A diglycidyl methacrylate, 1,3-butanediol dimethacrylate, ethylene glycol dimethacrylate (hereinafter referred to as 1G), diethylene glycol dimethacrylate (hereinafter referred to as 2G) and triethylene glycol dimethacrylate (hereinafter referred to as 3G) or mixtures of two or more thereof may be exemplified. In particular, as 3G is harmless to a living body and easily attaches to bones, it is favored.

In addition, when a composition comprising the thermoset resin and hydroxyapatite is used, since the thermoset resin retains hydroxyapatite and has excellent biocompatibility, and since hydroxyapatite has excellent biocompatibility, and, in particular, osteogenesis, the presence of hydroxyapatite gives the implant material even more excellent biocompatibility.

A layer made singly of the thermoset resin has excellent hardness and adhesion to the metallic material.

Accordingly, as to whether the layer is made singly of the thermoset resin or of the resin and hydroxyapatite, consideration should be given to the place of use, the use conditions and the shape in use of the material. The weight ratio of the thermoset resin to hydroxyapatite is generally from 65:35 to 15:85.

The hydroxyapatite is a synthetic or natural hydroxyapatite, a calcined product thereof or a mixture thereof.

As synthetic hydroxyapatite, those available by known processes of production, for instance, a dry process disclosed in "CERAMICS", Vol. 10(7), page 461 (1975) wherein $Ca_3(PO_4)_2$ and an excess of $CaCO_3$ are reacted in a water vapour flow at 900 to 1300°C, a wet process disclosed in JP-A-56/45814 wherein microparticles of calcium hydroxide are reacted with an aqueous solution of phosphoric acid with high speed stirring and a wet process disclosed in "Angewandte Chem., 67, page 327 (1955) wherein an aqueous solution of $Ca(NO_3)_2$ and an aqueous solution of $(NH_4)_2HPO_4$ are reacted under $NH_4OH$-alkaline conditions, and the calcined product thereof may be exemplified.

Natural hydroxyapatite can be produced from natural bones, for instance by calcining the backbone of a cow or ox at a temperature around $800°C$ and removing organic material. Hydroxyapatite may, if necessary, contain whitlockite or a foreign element such as fluorine or iron.

The particle grain size of the hydroxyapatite is generally smaller than 1000 micrometers in mean diameter, preferably from 0.01 to 100 micrometers from the view points of processability, handling and mechanical strength.

The metallic material includes all known metals and alloys. However, alloys of the Co-Cr-Ni and Co-Cr-Mo series, stainless steels 18-8, 316L, titanium and tantalum, which scarcely harm the tissue of a living body, have sufficient mechanical strength and is dense or porous material are preferable.

In order to obtain an anchor effect with the resin composition and to disperse the external stress after implanting, independent and/or continuous ditches may be installed on the outer surface of the metallic material.

The shape and size of the metallic material is not specifically limited; the shape can, for example, be pin-type, screw-type, blade-type, anchor-type, plate-type or mesh-type. The cross-section of the metallic material may be, for example, a square, circle, or oval.

To obtain the implant material according to the present invention, there is no limitation of method. For instance the implant material can be produced by (1) setting a core metallic material obtained by molding, sintering, casting, cutting or grinding and further subjecting it to surface treatment in a mold, (2) pouring a monomer of the thermosetting resin to which at least a hardening agent has been admixed preliminarily into the mold, (3) heating the core metallic material and the monomer in the mold and, after hardening the monomer, (4) cutting and grinding the outer surface of the thus hardened resin composition according to its use.

Following the conventional thermosetting method, internal strains are caused in the resin layer, thereby easily causing cracks on the interface to the core material. Accordingly, it is necessary to select carefully the conditions of hardening such as temperature and time. This problem can be overcome by adding powdery or granular thermosetting polymer to the monomer of the thermosetting resin.

The weight ratio of the polymer to the monomer from 5:95 to 95:5, preferably from 20:80 to 80:20. The shape of the thus added polymer is not particularly limited, and the mean particle diameter of the polymer is ordinarily from 2 to 500 $\mu$m, preferably from 10 to 200 $\mu$m.

The outer surface of the resin composition may be smooth or uneven, such as screw-like.

A suitable place in a human body for applying the implant material according to the present invention is not specifically limited. The implant material may, for example be applied within tooth and bone, under the periosteum and with the mucous membrane. The implant material may therefore be in the form of a piece of a tooth or bone.

When using the implant material according to the present invention as an artificial dental tooth root, there is no abnormal feeling because of the appropriate elastic modulus and hardness thereof.

On the outside of the resin composition which is positioned near the epiterium of the inner border, a structured body made singly of hydroxyapatite may be installed. In addition, the resin composition may be formed in the shape of a sword, thereby increasing adhesion to the epiterium.

The implant material according to the present invention is briefly explained in the Figures as follows.

In the vertical section shown in Figs. 1 and 3, the main body of the implant material according to the present invention (1 and 5) has a metallic core (3 and 7) coated by the resin composition (2 and 6).

For instance, the metallic core (3 and 7) of the main body of the implant material (1 and 5) is formed from an alloy of the titanium or cobalt series, and the outer surface of the core (3 and 7) is coated by a layer of a polymer of triethylene glycol dimethacrylate or a layer of a resin composition (2 and 6) made by adding 70% by weight of hydroxyapatite to 30% by weight of a polymer of triethylene glycol dimethacrylate. A screw-type groove (4 and 8) is provided on the core (3 and 7).

To the upper part of the implant material according to the present invention, a ready-made artificial dental crown of standard type, which has been freely selected, may be adhered and fixed, and the thus treated material may be used.

The present invention is further explained in the Examples as follows.

EXAMPLE 1:

Into a separable flask, 50 g of 3G monomer (made by MITSUBISHI Rayon Co., Ltd. under the name of ACRYLESTER (Registered Trade Mark) 3ED) and 50 g of 3G polymer of 23 $\mu$m mean particle diameter were introduced, and the contents of the flask were stirred at room temperature under vacuum to carry out deaeration thereof.

Then, 0.5% by weight of a hardening agent (t-butyl peroctoate) was added into the flask, and the contents of the flask were stirred for 30 minutes.

The thus prepared liquid resin was poured into a glass tube of 4 mm internal diameter in which a pure titanium stick 3 mm diameter and 100 mm long had been fixed. The liquid resin was subjected to a hardening treatment to obtain an implant material under the following conditions:

The liquid resin was heated for 5 hours at 55°C, 2 hours at 65°C, 1 hour at 70°C, 1 hour at 100°C, 30 min. at 110°C and then 30 min. at 120°C.

EXAMPLE 2:

Into a separable flask, 25 g of 3G monomer (same as in Example 1), 5 g of 3G polymer (same as in Example 1) and 70 g of hydroxyapatite of 4 µm average particle diameter were introduced, and the contents of the flask were stirred at room temperature under vacuum to carry out deaeration thereof.

Then, 0.3% by weight of the same hardening agent as in Example 1 (to 3G monomer) was added to the flask, and the contents of the flask were stirred fcr 30 minutes.

The thus prepared composition was poured into a glass tube of 5 mm inner diameter in which a pure titanium stick 3 mm diameter and 50 mm long had been fixed. Then, the glass tube was placed in an autoclave for dental use (manufactured by Shofu Dental Mfg. Co., Ltd.) filled with nitrogen gas at a starting pressure of 3 kg/cm² and the composition in the tube was subjected to hardening treatment by heating according to the schedule shown in Fig. 5.

EXAMPLE 3:

Each of the implant materials prepared in Examples 1 and 2 were processed into shapes 5 mm diameter and 15 mm long, and after grinding and smoothing the surface thereof, the thus processed implant materials were put into the alveolar bone of an adult dog.

By X-ray observation of the thus treated implanted materials, favorable bone-formations were confirmed without any abnormal findings, such as inflammation, 12 months after application.

## Claims

1. An implant material suitable for use in a living body, which material comprises a metallic material covered by a layer comprising a biocompatible, stable thermoset resin.

2. An implant material according to claim 1, wherein the resin layer further comprises hydroxyapatite .

3. An implant material according to claim 2, wherein the weight ratio of said thermoset resin to hydroxyapatite is from 65:35 to 15:85.

4. An implant material according to claim 2 or 3, wherein the hydroxyapatite is in the form of particles with a mean diameter smaller than 1000 um.

5. An implant material according to any one of the preceding claims, wherein said resin is a polymer of a monomer selected from bisphenol A diglycidyl methacrylate, 1,3-butanediol dimethacrylate, ethylene glycol dimethacrylate, diethylene glycol dimethacrylate, triethylene glycol dimethacrylate and mixtures of two or more thereof.

6. An implant material according to claim 5, wherein said resin is a polymer of triethylene glycol dimethacrylate.

7. An implant material according to any one of the preceding claims, wherein said thermoset resin has been prepared by mixing monomer therefor and from 5 to 95 wt. %, based on the weight of monomer, of polymer thereof and effecting hardening of the monomer.

8. An implant material according to claim 7, wherein the mean diameter of said polymer mixed with said monomer is 2-500 um.

9. An implant material according to any one of the preceding claims in the form of a piece of a tooth.

10. An implant material according to any one of the preceding claims in the form of a piece of a bone.

## Revendications

1. Matériau pour implant convenant pour emploi dans un corps vivant, matériau qui est constitué d'un matériau métallique recouvert d'une couche comprenant une résine thermodurcissable stable et biocompatible.

2. Matériau pour implant selon la revendication 1, dans lequel la couche de résine comprend en outre de l'hydroxyapatite.

3. Matériau pour implant selon la revendication 2, dans lequel le rapport, en poids, entre ladite résine thermodurcissable et l'hydroxyapatite va de 65:35 à 15:85.

4. Matériau pour implant selon la revendication 2 ou 3, dans lequel l'hydroxyapatite se présente sous la forme de particules d'un diamètre moyen inférieur à 1000 μm.

5. Matériau pour implant selon l'une quelconque des revendications précédentes, dans lequel ladite résine est un polymère d'un monomère choisi parmi le diglycidylméthacrylate du bisphénol A, le diméthylacrylate du 1,3-butanediol, le diméthylacrylate d'éthylèneglycol, le diméthylacrylate de diéthylèneglycol, le dyméthylactylate de triéthylèneglycol et des mélanges de deux ou plus de ces composants.

6. Matériau pour implant selon la revendication 5, dans lequel ladite résine est un polymère du dyméthylacrylate du triéthylèneglycol.

7. Matériau pour implant selon l'une quelconque des revendications précédentes, dans lequel on a préparé ladite résine thermodurcissable en mélangeant son monomère et de 5 à 95% en poids, sur la base du poids du monomère, de son polymère et en procédant au durcissement du monomère.

8. Matériau pour implant selon la revendication 7, dans lequel le diamètre moyen dudit polymère mélangé avec ledit monomère est de 2-500 μm.

9. Matériau pour implant selon l'une quelconque des revendications précédentes, se présentant sous forme d'un morceau de dent.

10. Matériau pour implant selon l'une quelconque des revendications précédentes se présentant sous forme d'un morceau d'os.

**Patentansprüche**

1. Implantatmaterial, geeignet zur Verwendung in einem lebenden Körper, enthaltend ein metallisches Material, das von einer Schicht bedeckt ist, welche ein biokompatibles stabiles hitzegehärtetes Harz enthält.

2. Implantatmaterial nach Anspruch 1, worin die Harzschicht zusätzlich Hydroxylapatit enthält.

3. Implantatmaterial nach Anspruch 2, worin das Gewichtsverhältnis des hitzegehärteten Harzes zum Hydroxylapatit 65:35 bis 15:85 beträgt.

4. Implantatmaterial nach Anspruch 2 oder 3, worin das Hydroxylapatit in Form von Teilchen mit einem mittleren Durchmesser kleiner als 1.000 μm vorliegt.

5. Implantatmaterial nach einem der vorhergehenden Ansprüche, worin das Harz ein Polymer eines Monomers ausgewählt aus Bisphenol A-Diglycidylmethacrylat, 1,3-Butandioldimethacrylat, Ethylenglycoldimethacrylat, Diethylenglycoldimethacrylat, Triethylenglycoldimethacrylat und Mischungen von zwei oder mehreren davon ist.

6. Implantatmaterial nach Anspruch 5, worin das Harz ein Polymer eines Triethylenglycoldimethacrylats ist.

7. Implantatmaterial nach einem der vorhergehenden Ansprüche, worin das hitzegehärtete Harz hergestellt wurde durch Mischen seines Monomers und 5 bis 95 Gew.%, bezogen auf das Gewicht des Monomers, eines Polymers davon und Härten des Monomers.

8. Implantatmaterial nach Anspruch 7, worin der mittlere Durchmesser des mit dem Monomer vermischten Polymers 2-500 μm beträgt.

9. Implantatmaterial nach einem der vorhergehenden Ansprüche in Form eines Zahnstückes.

10. Implantatmaterial nach einem der vorhergehenden Ansprüche in Form eines Knochenstückes.

Fig.1

Fig.2

Fig.3

Fig.4

# Fig.5